# EUROPÄISCHE PATENTSCHRIFT

(11) **EP 0 393 453 B1**
(45) Veröffentlichungstag und Bekanntmachung des Hinweises auf die Patenterteilung: **30.11.1994**
(21) Anmeldenummer: 90106721.5
(22) Anmeldetag: 07.04.1990
(51) Int. Cl.: C07D 213/61, C07D 213/64, C07D 213/26

(54) **Verfahren zur Herstellung von 2-Chlor-5-chlormethyl-pyridin und neue Zwischenprodukte**
Process for the preparation of 2-chloro-5-chloromethyl-pyridine and new intermediates
Procédé de préparation de pyridine-2-chloro-5-chlorométhyl et intermédiaires

(30) Priorität: 20.04.1989 DE 3912964
(43) Veröffentlichungstag der Anmeldung: 24.10.1990
(73) Patentinhaber: BAYER AG, 51368 Leverkusen (DE)
(72) Erfinder: Jelich, Klaus, Dr., D-5600 Wuppertal 1 (DE)

(56) Entgegenhaltungen:
- EP-A- 0 065 358
- EP-A- 0 163 855
- US-A- 2 695 902
- SYNTHESIS, September 1984, Seiten 743-746, Stuttgart, DE; T.R. KASTURI et al.:"A novel transformation of 3-alkoxyisoquinolines to 3-chloroisoquinolines andan unusual decyanation of 1,3-dialkoxy-4-cyano-5,6,7,8-tetrahydroisoquinolinesunder Vilsmeier-Haack conditions"
- Tetrahedron Letters, vol.25, no.49 (1984), pp.5693-6

## Beschreibung

Die Erfindung betrifft ein neues Verfahren zur Herstellung des als Zwischenprodukt für Insektizide bekannten 2-Chlor-5-chlormethyl-pyridins_{,} neue Zwischenprodukte hierfür und ein Verfahren zu deren Herstellung.

Es ist bekannt, daß man 2-Chlor-5-chlormethylpyridin in einem aufwendigen mehrstufigen Verfahren erhält, wenn man 2-Chlorpyridin-5-carbonsäure mit Thionylchlorid in das entsprechende Säurechlorid überführt, dieses gegebenenfalls mit Ethanol verestert, anschließend mit Natriumboranat zur Hydroxymethylverbindung reduziert und schließlich mit Thionylchlorid in der Seitenkette die Hydroxygruppe durch Chlor substituiert (vgl. z. B. US-PS 4 576 629; J. Heterocycl. Chem. 16, 333 - 337 [1979]).

Nachteilig bei diesem Verfahren und prohibitiv für eine großtechnische Durchführbarkeit ist jedoch der hohe Preis der Ausgangsverbindung 2-Chlorpyridin-5-carbonsäure und des Reduktionsmittels Natriumboranat, welches außerdem auch unter dem Aspekt der Wasserstofffreisetzung im Verlauf der Reaktion ein sicherheitstechnisches Problem darstellt.

Weiterhin ist bekannt, daß man 2-Chlor-5-chlormethylpyridin erhält, wenn man 2-Chlor-5-methylpyridin mit elementarem Chlor umsetzt (vgl. z. B. DE-A 36 30 046). Nachteilig bei diesem Verfahren ist jedoch, daß die Reaktion nicht einheitlich verläuft, so daß es nötig ist, um die Bildung von größeren Mengen an mehrfach chlorierten Nebenprodukten zu vermeiden, die Chlorierung frühzeitig abzubrechen, noch bevor die Umsetzung vollständig erfolgen konnte (vgl. auch EP-A 9 212; EP-A 65 358). Die entstehenden Gemische sind nur schwierig aufzutrennen und liefern Produkte mit nicht befriedigender Reinheit,

Aus der US-Patentschrift Nr. 2 695 902 ist die Umsetzung von Polyhydroxyalkoxyalkyl-substituierten Pyridinen mit Chlorierungsmitteln unter Druck bekannt.

Es wurde nun gefunden, daß man 2-Chlor-5-chlormethyl-pyridin der Formel (I) 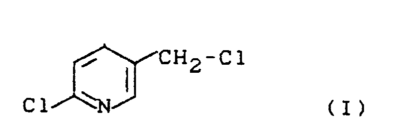
erhält, wenn man 2-Alkoxy-5-alkoxymethyl-pyridin-Derivate der allgemeinen Formel (II) 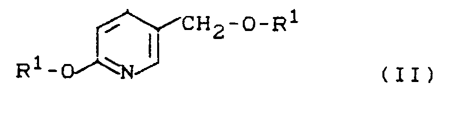
in welcher
- R¹: für Alkyl mit 1 bis 6 Kohlenstoffatomen steht,
mit einem Chlorierungsmittel der Reihe Phosphor(V)-chlorid, Phosphorylchlorid(Phosphoroxychlorid), oder Mischungen aus Phosphor(V)-chlorid und Phosphorylchlorid, gegebenenfalls in Gegenwart eines Verdünnungsmittels und gegebenenfalls in Gegenwart eines Reaktionshilfsmittels der Reihe tertiäre Amine, Formamide oder Metallhalognide bei Temperaturen zwischen 0°C und 200°C umsetzt.

Es ist als überraschend anzusehen, daß die Umsetzung von 2-Alkoxy-5-alkoxymethyl-pyridin-Derivaten der Formel (II) mit Chlorierungsmitteln gemäß dem erfindungsgemäßen Verfahren zu einem Austausch beider Alkoxygruppen gegen Chlor führt, da aus dem Stand der Technik bekannt war, daS für eine großtechnische Durchführung wenig geeignete "Vilsmeier-Haack-Bedingungen" (d. h. Phosphoroxychlorid in Gegenwart von großen Mengen Dimethylformamid, wobei bei der Aufarbeitung erhebliche Abwassermengen anfallen) für eine Überführung von 2-Methoxypyridin in 2-Chlor-pyridin erforderlich seien, wobei Phosphoroxychlorid in Abwesenheit von Dimethylformamid keinerlei Umsetzung bewirkte. Außerdem liefert diese Reaktion mit weniger als 40 % Ausbeute nur ein äußerst unsauberes Produkt, welches aufwendig chromatographisch gereinigt werden muß (vgl. hierzu Synthesis 1984, 743 - 745).

Vorteile des erfindungsgemäßen Verfahrens liegen in der guten Zugänglichkeit der als Ausgangsverbindungen benötigten 2-Alkoxy-5-alkoxymethyl-pyridin-Derivate (II) sowie in der insgesamt geringen Zahl der Synthesestufen und der Verwendung billiger Synthesechemikalien bei der Herstellung von (II) , wobei als Basisverbindung die billige Chemikalie 3-Methylpyridin eingesetzt werden kann.

Verwendet man beispielsweise 2-Methoxy-5-methoxymethyl-pyridin und Phosphor(V)-chlorid als Ausgangsstoffe, so kann der Reaktionsablauf beim erfindungsgemäßen Verfahren durch das folgende Formelschema skizziert werden: 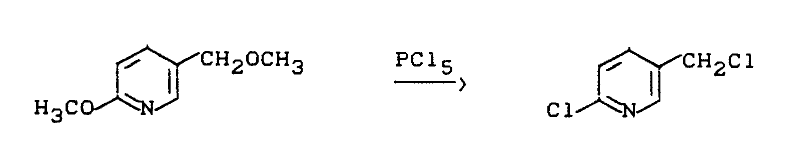
Die als Ausgangsstoffe zu verwendenden 2-Alkoxy-5-alkoxymethyl-pyridin-Derivate sind durch die Formel (II) allgemein definiert. In Formel (II) steht R¹ für geradkettiges oder verzweigtes Alkylmit 1 bis 6 Kohlenstoffatomen, vorzugsweise mit 1 bis 4 Kohlenstoffatomen.

Als Beispiele für die Ausgangsstoffe der Formel (II) seien genannt:
2-Methoxy-5-methoxymethyl-pyridin, 2-Ethoxy-5-ethoxymethyl-pyridin, 2-Propoxy-5-propoxymethyl-pyridin, 2-Isopropoxy-5-isopropoxymethyl-pyridin, 2-Butoxy-5-butoxymethyl-pyridin, 2-Isobutoxy-5-isobutoxymethyl-pyri-din, 2-sec-Butoxy-5-sec-butoxymethyl-pyridin und 2-tert-Butoxy-5-tert-butoxymethyl-pyridin.

Die Ausgangsstoffe der Formel (II) sind noch nicht bekannt. Man erhält die neuen 2-Alkoxy-5-alkoxymethyl-pyridin-Derivate der Formel (II), wenn man 3-Dichlor-methyl-pyridin der Formel (III) 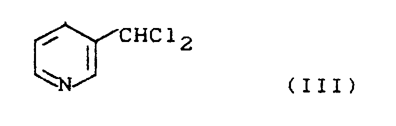
mit einem Alkohol der Formel (IV)

R¹OH (IV)

worin
- R¹: die oben angegebene Bedeutung hat,
und mit Alkalimetallsalzen von Alkoholen der Formel (IV)
bei Temperaturen zwischen 0°C und 150°C, vorzugsweise zwischen 20°C und 100°C, umsetzt und nach üblichen Methoden aufarbeitet. So wird beispielsweise nach erfolger Umsetzung das Reaktionsgemisch eingeengt, nach dem Abkühlen mit organischen Lösungsmittel (z.B. Ether) verrührt, Salze durch Absaugen abgetrennt und das Filtrat eingeengt. Der Rückstand wird dann nach üblichen Methoden, z.B. durch Chromatographie gereinigt.

Für den vorausgehend beschriebenen Weg zur Herstellung der neuen 2-Alkoxy-5-alkoxymethyl-pyridin-Derivate ist aus der Chemie der Pyridinderivate kein Vorbild bekannt. Es ist somit als überraschend anzusehen, daß aus 3-Di-chlormethyl-pyridin und Alkoholaten 2-Alkoxy-5-alkoxymethyl-pyridine hergestellt werden können.

Aus der Publikation Tetrahedron Letters, Vol 25, No. 29 (1984), pp 5693-5696, ist die Umsetzung der die stark elektronenziehende Trichlormethylgruppe enthaltenden Pyridine mit Natriummethylat im Methanol bekannt.

Das als Zwischenprodukt benötigte 3-Dichlormethylpyridin der Formel (III) ist bereits bekannt (vgl. EP-A 9212 und EP-A 65358), wird jedoch bei der Chlorierung von 3-Methylpyridin im allgemeinen als Nebenprodukt erhalten.

Es wurde nun weiterhin gefunden, daß man 3-Dichlor-methylpyridin der Formel (III) in guten Ausbeuten als Hauptprodukt erhält, wenn man 3-Methylpyridin der Formel (V) 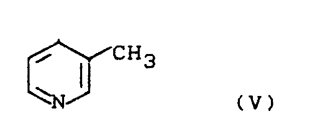
mit elementaren Chlor in Gegenwart einer organischen Säure, wie z.B. Essigsäure, Propionsäure, Chloressigsäure, Dichloressigsäure, Trichloressigsäure, Trifluoressigsäure, Methansulfonsäure und/oder Trifluormethansulfonsäure (vorzugsweise Essigsäure) und gegebenenfalls zusätzlich in Gegenwart einer anorganischen Säure, wie z.B. Hydrogenchlorid, Hydrogenbromid oder Schwefelsäure (vorzugsweise Schwefelsäure), sowie in Gegenwart eines Radikalstarters, wie z.B. Azo-bis-isobutyronitril, Benzoylperoxid oder tert-Butyl-perbenzoat, bei Temperaturen zwischen -20°C und +150°C, vorzugsweise zwischen 0°C und 100°C, umsetzt und nach üblichen Methoden aufarbeitet, z.B. durch Aufnahme des Reaktionsproduts in einem geeigneten organischen Lösungsmittel, wie z.B. Essigsäureethylester, Neutralisation mit Lauge (z.B. NaOH), Abtrennung der organischen Phase, Trocknung der organischen Phase und Abdestillieren des Lösungsmittels.

In Anbetracht des Standes der Technik (Verbindung (III) fällt dort nur als Nebenprodukt an) ist der selektive Verlauf des vorausgehend beschriebenen Verfahrens als ausgesprochen überraschend zu bezeichnen.

Die weiter als Ausgangsstoffe benötigten Alkohole sind durch die Formel (IV) allgemein definiert. In der Formel (IV) steht R¹ für geradkettiges oder verzweigtes Alkyl, vorzugsweise mit 1 bis 4 Kohlenstoffatomen.

Es werden bei der Herstellung von Verbindungen der Formel (II) vorzugsweise die Lithium-, Natrium- oder Kalium-salze, insbesondere die Natriumsalze dieser Alkohole eingesetzt.

Als Beispiele seien genannt:
Methanol, Ethanol, Propanol, Isopropanol, Butanol, Isobutanol, sec-Butanol und tert-Butanol sowie die Natriumsalze dieser Alkohole,

Bei der Herstellung von 2-Chlor-5-chlormethyl-pyridin der Formel (I) aus 2-Alkoxy-5-alkoxymethyl-pyridin-Derivaten der Formel (II) nach dem erfindungsgemäßen Verfahren kommen als Chlorierungsmittel Phosphor(V)-chlorid, Phosphorylchlorid (Phosphoroxychlorid), insbesondere Mischungen aus Phosphor(V)-chlorid und Phosphorylchlorid in Betracht.

Das erfindungsgemäße Verfahren kann entweder ohne Zusatz eines Verdünnungsmittels in Substanz oder in Gegenwart eines geeigneten Verdünnungsmittels durchgeführt werden. Hierzu gehören insbesondere aliphatische, alicyclische oder aromatische, gegebenenfalls halogenierte Kohlenwasserstoffe, wie beispielsweise Benzol, Toluol, Xylole, Chlorbenzol, Dichlorbenzole, Petrolether, Hexan, Cyclohexan, Methylcyclohexan, Dichlormethan, Chloroform oder Tetrachlormethan.

Das erfindungsgemäße Verfahren wird gegebenenfalls in Gegenwart eines Reaktionshilfsmittels der Reihe tertiäre Amine, wie Triethylamin, N,N-Dimethyl-anilin, Pyridin oder 4-Dimethylamino-pyridin, katalytische Mengen an Formamiden, wie N,N-Dimethyl-formamid oder N,N-Dibutyl-formamid, oder Metallhalogenide wie Magnesiumchlorid oder Lithiumchlorid durchgeführt.

Die Reaktionstemperaturen können bei der Durchführung des erfindungsgemäßen Verfahrens in einem größeren Bereich variiert werden. Im allgemeinen arbeitet man bei Temperaturen zwischen 0°C und 200°C, vorzugsweise zwischen 10°C und 120°C.

Zur Durchführung des erfindungsgemäßen Verfahrens setzt man auf 1 Mol 2-Alkoxy-5-alkoxymethyl-pyridin-Derivat der Formel (II) im allgemeinen zwischen 1 und 10 Mol-äquivalenten, vorzugsweise zwischen 1 und 5 Moläquivalenten, des Chlorierungsmittels ein.

Die Reaktionskomponenten werden im allgemeinen unter leichtem Kühlen vermischt und dann im allgemeinen bei erhöhter Temperatur bis zum Ende der Umsetzung gerührt. Die Aufarbeitung erfolgt nach üblichen Methoden (vgl. Herstellungsbeispiele).

Das nach dem erfindungsgemäßen Verfahren erhältliche 2-Chlor-5-chlormethyl-pyridin der Formel (I) kann als Zwischenprodukt für die Herstellung von Insektiziden verwendet werden (vgl. EP-A 163855, EP-A 192060).

### Herstellungsbeispiele

### Beispiel 1

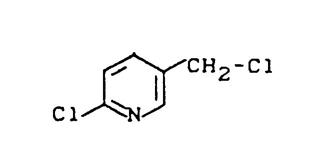

Zu 5,1 g (33,3 mMol) Phosphoroxychlorid gibt man zunächst 14 g (66,6 mMol) Phosphor(V)-chlorid und anschließend unter Kühlen mit einem Eisbad portionsweise 5,1 g (33,3 mMol) 2-Methoxy-5-methoxymethyl-pyridin. Das Reaktionsgemisch wird 3 Stunden unter Rückfluß zum Sieden erhitzt und anschließend im Vakuum eingeengt. Der Rückstand wird mit Eiswasser verdünnt, mit 2N-Natronlauge neutralisiert und mit Essigsäureethylester geschüttelt, Die organische Phase wird abgetrennt, mit Natriumsulfat getrocknet und filtriert. Vom Filtrat wird das Lösungsmittel im Wasserstrahlvakuum abdestiliert. Man erhält 2,4 g (45% der Theorie) 2-Chlor-5-chlormethyl-pyridin als Rückstand (gelbe Flüssigkeit).

### Ausgangsstoffe der Formel (II)

### Beispiel (II-1)

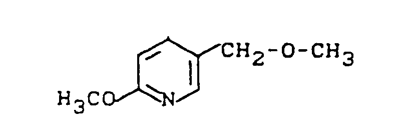

Eine Lösung von 35,8 g (73%ig; 0,161 Mol) 3-Dichlormethyl-pyridin in 50 ml Methanol wird zu einer unter Rückfluß zum Sieden erhitzten Lösung von 29,5 g (0,55 Mol) Natrium-methylat in 90 ml Methanol tropfenweise gegeben. Das Reaktionsgemisch wird weitere 4 Stunden unter Rückfluß zum Sieden erhitzt und anschließend eingeengt.

Der Rückstand wird mit Diethylether verrührt, das ungelöst gebliebene Natriumchlorid durch Absaugen abgetrennt und das Filtrat wird eingeengt. Der Rückstand wird durch Chromatographie an Kieselgel (Laufmittel: Petrolether/Essigsäureethylester, Essigsäureethylester, Vol. 5:1) gereinigt.

Man erhält 12,3 g (50% der Theorie) 2-Methoxy-5-methoxymethyl-pyridin.

¹H-NMR (CDCl₃, δ, ppm): 2,8; 3,9; 4,4.

Analog erhält man

### Beispiel (II-2)

aus 3-Dichlormethylpyridin mit Natriumethylat und Ethanol die Verbindung: 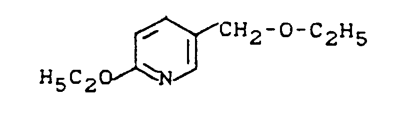
¹H-NMR (CDCl₃, δ, ppm): 3,5; 4,3; 4,4.

### Beispiel (II-3)

aus 3-Dichlormethylpyridin mit Natriumisopropylat und Isopropanol die Verbindung: 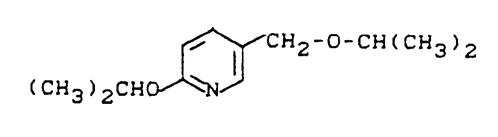
¹H-NMR (CDCl₃, δ, ppm): 4,4.

### Beispiel (II-4)

aus 3-Dichlormethylpyridin mit Natriumisobutylat und Isobutanol die Verbindung: 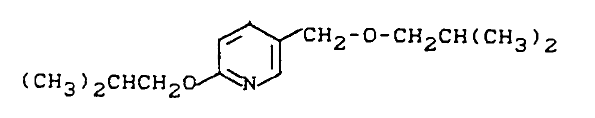
¹H-NMR (CDCl₃, δ, ppm): 3,2; 4,05; 4,4.

### Ausgangsverbindung der Formel (III)

### Beispiel (III-1)

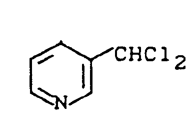

23,3 g (0,25 Mol) 3-Methylpyridin werden unter Rühren zu einer mit einem Eisbad gekühlten Mischung aus 25 g (0,255 Mol) konz. Schwefelsäure und 150 ml Essigsäure tropfenweise gegeben. Die Reaktionsmischung wird auf 75°C erhitzt und unter Durchleiten eines kräftigen Chlorstroms wird eine Lösung von 4,0 g Azo-bisisobutyronitril in 30 ml Eisessig innerhalb von 8 Stunden tropfenweise dazugegeben. Nach Einengen wird der Rückstand in Essigsäureethylester aufgenommen und mit 2N-Natronlauge neutralisiert. Die organische Phase wird abgetrennt, mit Natriumsulfat getrocknet und filtriert. Vom Filtrat wird das Lösungsmittel im Wasserstrahlvakuum abdestilliert. Man erhält 41 g (73% der Theorie, 79%ig) 3-Dichlormethyl-pyridin als Rückstand (gelbe Flüssigkeit).

## Patentansprüche

1. Verfahren zur Herstellung von 2-Chlor-5-chlormethyl-pyridin der Formel (I) 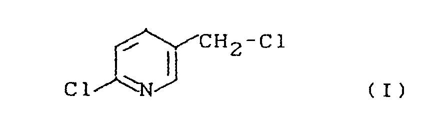 dadurch gekennnzeichnet, daß man 2-Alkoxy-5-alkoxy-methylpyridin-Derivate der allgemeinen Formel (II) 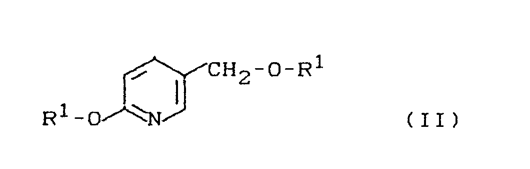 in welcher
R¹ für Alkyl mit 1 bis 6 Kohlenstoffatomen steht,
mit einem Chlorierungsmittel der Reihe Phosphor(V)-chlorid, Phosphorylchlorid (Phosphoroxyclorid), oder Mischungen aus Phosphor(V)-chlorid und Phosphorylchlorid, gegebenenfalls in Gegenwart eines Verdünnungsmittels aus der Reihe aliphatischer, alicyclischer, aromatischer, gegebenenfalls halogenierter Kohlenwasserstoffe und gegebenenfalls in Gegenwart eines Reaktionshilfsmittels der Reihe tertiäre Amine, katalytische Mengen an Formamiden oder Metallhalogenide bei Temperaturen zwischen 0°C und 200°C umsetzt.

2. Verfahren gemäß Anspruch 1, dadurch gekennzeichnet, daß zwischen 10°C und 120°C gearbeitet wird.

3. Verfahren gemäß Anspruch 1-2, dadurch gekennzeichnet, daß man pro Mol 2-Alkoxy-5-alkoxymethylpyridin-Derivat der Formel (II) 1 bis 10 Moläquivalente des Chlorierungsmittels einsetzt.

4. 2-Alkoxy-5-alkoxymethyl-pyridin-Derivate der allgemeinen Formel (II) 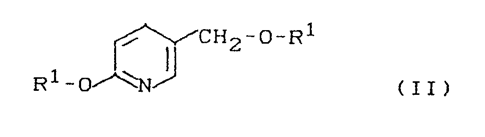 in welcher
der Rest R¹ für Alkyl mit 1 bis 6 Kohlenstoffatomen steht.

5. Verfahren zur Herstellung von 2-Alkoxy-5-alkoxy-methylpyridin-Derivaten der allgemeinen Formel (II) 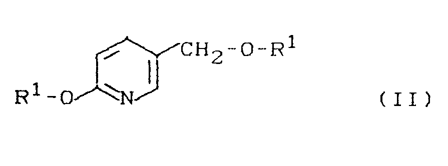 in welcher
R¹ für Alkyl mit 1 bis 6 Kohlenstoffatomen steht,
dadurch gekennzeichnet, daß man 3-Dichlormethyl-pyridin der Formel III 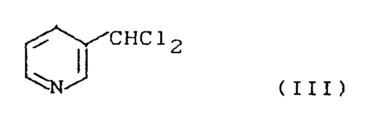 mit einem Alkohol der Formel R¹-OH (IV) und mit dem entsprechenden Alkalimetallalkoholat dieses Alkohols bei Temperaturen zwischen 0°C und 150°C umsetzt.

6. Verfahren zur Herstellung von 3-Dichlormethyl-pyridin der Formel (III) 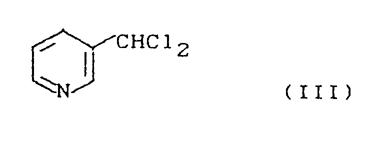 dadurch gekennzeichnet, daß man 3-Methylpyridin der Formel (V) 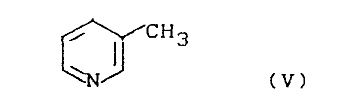 mit elementarem Chlor in Gegenwart einer organischen Säure und gegebenenfalls zusätzlich in Gegenwart einer anorganischen Säure sowie in Gegenwart eines Radikalstarters bei Temperaturen zwischen -20°C und +150°C umsetzt.

7. Verfahren gemäß Anspruch 6, dadurch gekennzeichnet, daß man als Radikalstarter eine Verbindung der Reihe Azo-bis-isobutyronitril, Benzoylperoxid, tert.-Butyl-perbenzoat, einsetzt und die Reaktion bei Temperaturen zwischen 0°C und 100°C durchführt.

## Claims

1. Process for the preparation of 2-chloro-5-chloro-methyl-pyridine, of the formula (I) 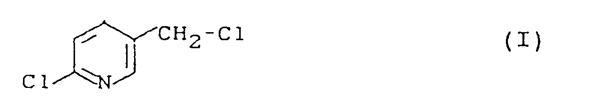 characterized in that 2-alkoxy-5-alkoxymethylpyridine derivatives of the general formula (II) 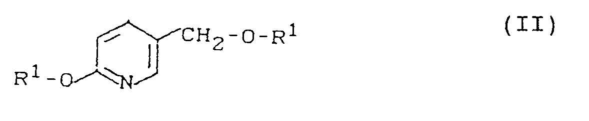 in which
R¹ represents alkyl having 1 to 6 carbon atoms,
are reacted with a chlorinating agent from the series comprising phosphorus(V) chloride, phosphoryl chloride (phosphorus oxychloride), or mixtures of phosphorus(V) chloride and phosphoryl chloride, if appropriate in the presence of a diluent from the series comprising aliphatic, alicyclic, aromatic, optionally halogenated hydrocarbons and if appropriate in the presence of a reaction auxiliary from the series comprising tertiary amines, catalytic amounts of formamides or metal halides, at temperatures between 0°C and 200°C.

2. Process according to Claim 1, characterized in that the process is carried out between 10°C and 120°C.

3. Process according to Claims 1-2, characterized in that 1 to 10 mole equivalents of the chlorinating agent are employed per mole of 2-alkoxy-5-alkoxymethylpyridine derivative of the formula (II).

4. 2-Alkoxy-5-alkoxymethyl-pyridine derivatives of the general formula (II) 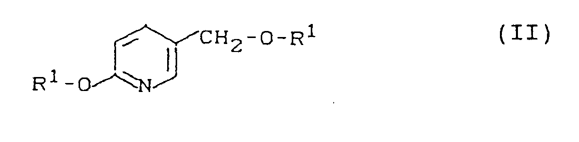 in which
the radical R¹ represents alkyl having 1 to 6 carbon atoms.

5. Process for the preparation of 2-alkoxy-5-alkoxymethylpyridine derivatives of the general formula (II) 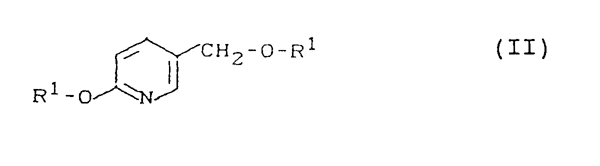 in which
R¹ represents alkyl having 1 to 6 carbon atoms,
characterized in that 3-dichloromethylpyridine, of the formula III, 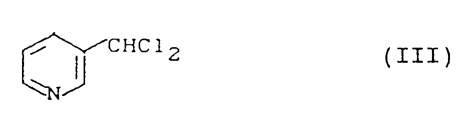 is reacted with an alcohol of the formula R¹-OH (IV) and with the corresponding alkali metal alkoxide of this alcohol, at temperatures between 0°C and 150°C.

6. Process for the preparation of 3-dichloromethyl-pyridine of the formula III) 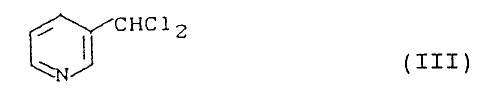 characterized in that 3-methylpyridine, of the formula (V), 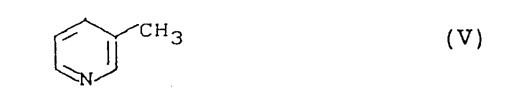 is reacted with elemental chlorine in the presence of an organic acid and if appropriate additionally in the presence of an inorganic acid and also in the presence of a free-radical initiator, at temperatures between -20°C and +150°C.

7. Process according to Claim 6, characterized in that a compound of the series comprising azo-bis-isobutyronitrile, benzoyl peroxide and tert.-butyl perbenzoate is employed as the free-radical initiator, and in that the reaction is carried out at temperatures between 0°C and 100°C.

## Revendications

1. Procédé de production de 2-chloro-5-chloro-méthylpyridine de formule (I) 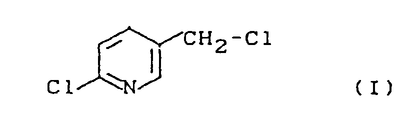 caractérisé en ce qu'on fait réagir à des températures comprises entre 0°C et 200°C des 2-alkoxy-5-alkoxyméthyl-pyridines de formule générale (II) 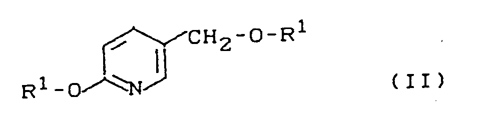 dans laquelle
R¹ est un groupe alkyle ayant 1 à 6 atomes de carbone,
avec un agent de chloration de la série du chlorure de phosphore (V), du chlorure de phosphoryle (oxychlorure de phosphore) ou de mélanges de chlorure de phosphore (V) et de chlorure de phosphoryle, le cas échéant en présence d'un diluant de la série d'hydrocarbures aliphatiques, alicycliques ou aromatiques éventuellement halogénés et le cas échéant en présence d'une substance auxiliaire de réaction de la série d'amines tertiaires, de quantités catalytiques de formamides ou d'halogénures métalliques.

2. Procédé suivant la revendication 1, caractérisé en ce qu'on opère entre 10°C et 120°C.

3. Procédé suivant les revendications 1 et 2, caractérisé en ce qu'on utilise 1 à 10 équivalents molaires de l'agent de chloration par mole de 2-alkoxy-5-alkoxyméthyl-pyridine de formule (II).

4. 2-alkoxy-5-alkoxyméthylpyridines de formule générale (II) 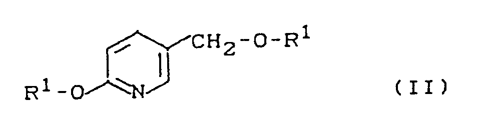 dans laquelle
le reste R¹ est un reste alkyle ayant 1 à 6 atomes de carbone.

5. Procédé de production de 2-alkoxy-5-alkoxyméthylpyridines de formule générale (II) 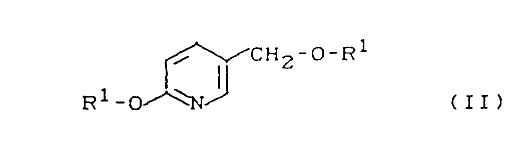 dans laquelle
R¹ est un groupe alkyle ayant 1 à 6 atomes de carbone,
caractérisé en ce qu'on fait réagir à des températures comprises entre 0°C et 150°C la 3-dichlorométhylpyridine de formule (III) 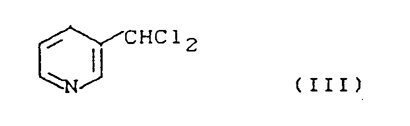 avec un alcool de formule R¹-OH (IV) et avec l'alcoolate de métal alcalin correspondant de cet alcool.

6. Procédé de production de 3-dichlorométhylpyridine de formule (III) 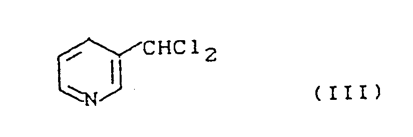 caractérisé en ce qu'on fait réagir à des températures comprises entre -20°C et +150°C la 3-méthylpyridine de formule (V) 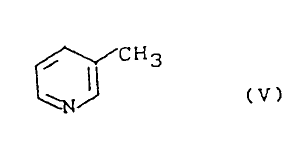 avec du chlore élémentaire en présence d'un acide organique et en outre, le cas échéant, en présence d'un acide inorganique de même qu'en présence d'un générateur de radicaux.

7. Procédé suivant la revendication 6, caractérisé en ce qu'on utilise comme générateur de radicaux un composé de la série de l'azo-bis-isobutyronitrile, du peroxyde de benzoyle et du perbenzoate de tertio-butyle, et on conduit la réaction à des températures comprises entre 0°C et 100°C.
